# EUROPEAN PATENT APPLICATION

(11) **EP 4 763 219 A1**
(43) Date of publication of application: **24.06.2026**
(21) Application number: 24222944.1
(22) Date of filing: 23.12.2024
(51) Int. Cl.: A61K 39/04, A61P 11/00, A61P 31/04, A61K 39/00

(54) **RECOMBINANT MYCOBACTERIUM AS A VACCINE IN THE PREVENTION OF TUBERCULOSIS OCCURRENCE AND RECURRENCE**

(71) Applicant: Serum Institute of India Private Limited, Pune 411 028 MAH (IN); Serum Life Science Europe GmbH, 30659 Hannover (DE)
(72) Inventor: SHALIGRAM, Umesh, 411028 Hadapsar Pune (IN); GRODE, Leander, 30659 Hannover (DE)
(74) Representative: Weickmann & Weickmann PartmbB

(57) **Abstract**

The invention relates to a recombinant Mycobacterium cell for use as a safe and effective vaccine in the prevention of tuberculosis occurrence or recurrence.

## Description

The invention relates to a recombinant Mycobacterium cell for use as a safe and effective vaccine in the prevention of tuberculosis occurrence or recurrence.

### Background

With 10.6 million annual cases and 1.3 million annual deaths, tuberculosis (TB) remains one of the most important threatening global health problems.' Around 25% of global TB cases (2.77 million) and deaths (0.36 million) occurred in India in 2022² while about one fourth (22.6%) of the individuals are infected with TB.³ In Bangladesh, there were an estimated 375,000, TB cases and 43,000 deaths in 2021.⁴

Patients with drug-susceptible TB receive an initial two-month intensive treatment followed by a four-month continuation phase with a daily dosing regimen of therapy.⁵ In India, the percentage of smear-positive re-treatment cases out of all smear-positive cases is 24%.⁶ The causes of re-treatment include recurrence, treatment failure and default in treatment.⁶

Patients with drug-susceptible tuberculosis receive an initial two-month intensive treatment followed by a four-month continuation phase with a daily dosing regimen of therapy.⁵ In India, the percentage of smear-positive re-treatment cases out of all smear-positive cases is 24%.⁶ The causes of re-treatment include recurrence, treatment failure and default in treatment.⁶

There are considerable variations in recurrence rates from 0 to 14% in studies from around the world.⁷ Studies from India have shown relapse rates from 6.5% to 12.3% based on the definition and the type of follow-up used.^{7,8,9} The majority of recurrence cases occur soon after completion of treatment, mostly in the first six months. Risk factors for recurrence include drug irregularity, initial drug resistance, smoking and alcoholism.^{10,11} The comparison of intermittent versus daily treatment has shown variable results.^{12,13,14,15} India introduced daily regimen in November 2017 in phase-wise manner.¹⁶

With 10.6 million annual primary TB cases,¹ TB recurrence represents a significant obstacle, especially in low-middle income countries (LMIC). The majority of recurrent TB cases are due to relapse rather than re-infection.¹⁷ Further, recurrence also increase the number of multidrug-resistant (MDR) TB cases.¹⁸ In addition, recurrence rates in patients infected with MDR *M. tuberculosis* (Mtb) strains are higher compared to those with drug-susceptible strains.⁸

Currently, there is no vaccine licensed for prevention of recurrent TB. Since BCG has inadequate efficacy against TB,¹⁹ there is an ongoing drive to develop more effective vaccines, notably for adolescents and adults.

A previous study for prevention of recurrent TB with a TB vaccine candidate has failed. H56:IC31, a TB candidate was recently tested for prevention of TB recurrence in a Phase-2b study. However, the vaccine did not provide protection against TB recurrence and in fact, possibly increased risk of recurrence among people who received the study vaccine (5.8%) compared to people who received the placebo (3.4%).³²

VPM1002, a new genetically modified BCG vaccine, harnesses the MHC-II pathway to present peptides by antigen-presenting cells (APCs) after vaccination, resulting in preferential stimulation of CD4+ helper T cells with weak stimulation of CD8+ cytotoxic T cells.^{20.21.22} Therefore, VPM1002 was generated in order to direct mycobacterial antigens to the MHC I pathway in addition to the MHC II pathway.^{19,23}

VPM1002 was found effective not only as a pre- but also as a post-exposure vaccine against relapse in a mouse model.^{23,24} Clinical studies have shown that VPM1002 is safe and well tolerated and immunogenic in humans; it induced multifunctional CD4+ and CD8+ T cell subsets. The immunogenicity of VPM1002 as detected by IFN-γ release by stimulated T cells was dose dependent.^{25,26,27}

In view of the previous negative results of other TB vaccine candidates, it could not have been expected that VPM1002 would be an effective vaccine for the prevention of recurrent TB.

### Summary of the Invention

In the present study, the inventors assessed the efficacy of VPM1002 in prevention of recurrent TB in patients who recovered from TB disease.

Surprisingly, they found that VPM1002 was efficacious against symptomatic, bacteriologically confirmed and clinically diagnosed TB recurrence, especially in the youngest age group of patients between 18 to 49 years. Further, the vaccine was found safe and well tolerated.

These results demonstrate VPM1002 is a safe and effective vaccine for the prevention of TB recurrence.

Further, the results of the study make it plausible that VPM1002 is particularly effective in younger human subjects, more particularly in adults of 18 to 49 years and also in adolescents, e.g., subjects between 6 and 17 years.

Thus, a first aspect of the present invention is a live Mycobacterium cell, which comprises a recombinant nucleic acid molecule encoding a fusion polypeptide comprising:
(a) a domain capable of eliciting an immune response, and
(b) a phagolysosomal escape domain
for use in a method, wherein the recombinant BCG cell is administered as a vaccine against TB to a human subject which has previously been subjected to a drug treatment against TB.

This first aspect of the present invention also relates to a method of vaccinating a human subject against TB, comprising administering to said subject an effective amount of a live Mycobacterium cell which comprises a recombinant nucleic acid molecule encoding a fusion polypeptide comprising:
(a) a domain capable of eliciting an immune response, and
(b) a phagolysosomal escape domain,
wherein the human subject has previously been subjected to a drug treatment against TB.

A second aspect of the present invention is a live Mycobacterium cell, which comprises a recombinant nucleic acid molecule encoding a fusion polypeptide comprising:
(a) a domain capable of eliciting an immune response, and
(b) a phagolysosomal escape domain
for use in a method, wherein the recombinant BCG cell is administered as a vaccine against TB to a human subject which is between 6 and 49 years.

This second aspect of the present invention also relates to a method of vaccinating a human subject against TB, comprising administering to said subject an effective amount of a live Mycobacterium cell which comprises a recombinant nucleic acid molecule encoding a fusion polypeptide comprising:
(a) a domain capable of eliciting an immune response, and
(b) a phagolysosomal escape domain,
wherein the human subject is between 6 and 49 years.

In particular embodiments of the above aspects, the live Mycobacterium cell is a recombinant urease-deficient *M. bovis* BCG cell from the Danish family strain.

### Detailed Description

The present invention is based on a double-blind randomized controlled study conducted in India and Bangladesh. The study population was bacteriologically confirmed pulmonary TB patients (age 18 to 65 years) who had previously been successfully treated and cured. Within a month of the treatment completion, they were randomized 1:1 to receive single dose of either VPM1002 or placebo. The primary outcome was bacteriologically confirmed TB recurrence cases during period 2 to 12 months after study vaccination in order to calculate the vaccine efficacy (VE). *Post-hoc* VE was also calculated for symptomatic bacteriologically confirmed and clinically diagnosed cases in age group of 18 to 49 years. Solicited reactions were collected for 2 months after vaccination while unsolicited adverse events (AEs) and serious adverse events (SAEs) were collected throughout the study.

The study enrolled 2001 cured pulmonary TB patients during January 2018 to December 2022. There were 29 cases of bacteriologically confirmed TB recurrence in the vaccine group and 31 in placebo group giving a VE of 7.8% (NE, 45.1). For symptomatic bacteriologically confirmed TB recurrence cases the VE was 25.4% (NE, 59.4). For bacteriologically confirmed symptomatic TB recurrence cases in age group 18 to 49 years, the VE was 34.0% (NE, 66.7). VE for symptomatic overall TB recurrence (bacteriologically confirmed as well as clinically diagnosed) cases was 31.0% (NE, 62.0) and the same in the age group 18 to 49 years was 39.9% (NE, 69.4). The incidence of local reactions including ulcer and scar formation was higher in the vaccine group as compared to the placebo group. The vaccine did not cause any SAEs.

To conclude, VPM1002 was found very safe and was well tolerated in the cured TB patients. The vaccine also showed a strong trend towards prevention of symptomatic TB recurrence, especially in the younger subjects, despite the challenges of testing TB vaccine in this indication and overcoming the natural immunity caused by MTB infection. The present study indicates that VPM1002 can be considered as an option to prevent TB recurrence. Further, the present study indicates that VPM1002 is especially effective in younger human subjects.

The vaccine of the present invention is a live recombinant Mycobacterium cell which comprises a recombinant nucleic acid molecule encoding a fusion polypeptide comprising (a) a domain capable of eliciting an immune response and (b) a phagolysosomal escape domain. The domain capable of eliciting an immune response is preferably an immunogenic peptide or polypeptide from a pathogen or an immunogenic fragment thereof.

The Mycobacterium cell is preferably an *M. bovis* cell, an *M. tuberculosis* cell, particularly an attenuated *M. tuberculosis* cell or another Mycobacterium cell, e.g. *M. microti, M. smegmatis, M. canettii, M. marinum* or *M. fortuitum.* More preferably, the cell is an attenuated recombinant *M. bovis* cell, particularly an *M. bovis* BCG cell, more particularly a recombinant *M. bovis* BCG cell from the Danish family strain (Brosch et al., Proc. Natl. Acad. Sci. USA, 104 (2007), 5396-5601).

In an especially preferred embodiment, the Mycobacterium cell is recombinant urease-deficient. In an especially preferred embodiment, the *ureC* sequence of the Mycobacterium cell is inactivated (Δ*UreC*), e.g., by constructing a suicide vector containing a *ureC* gene disrupted by a selection marker gene, e.g., the hygromycin gene, transforming the target cell with the vector and screening for selection marker-positive cells having a urease negative phenotype. In an even more preferred embodiment, the selection marker gene, i.e., the hygromycin gene, is subsequently inactivated. In this embodiment, the cell is a selection marker-free urease-deficient recombinant Mycobacterium cell.

The domain capable of eliciting an immune response is preferably selected from immunogenic peptides or polypeptides from *M. bovis, M. tuberculosis* or *M. leprae* or from immunogenic fragments thereof having a length of at least 6, preferably at least 8 amino acids, more preferably at least 9 amino acids and e.g., up to 20 amino acids. Specific examples for suitable antigens are Ag85B (p30) from *M. tuberculosis,* Ag85B (α-antigen) from *M. bovis* BCG, Ag85A from *M. tuberculosis* and ESAT-6 from *M*. *tuberculosis* and fragments thereof. In other embodiments, the domain capable of eliciting an immune response is selected from non-Mycobacterium polypeptides.

More preferably, the immunogenic domain is derived from the antigen Ag85B. Most preferably, the immunogenic domain comprises the sequence from aa. 41 to aa.51 in SEQ ID NO. 2.

The recombinant nucleic acid molecule further comprises a phagolysosomal escape domain, i.e., a polypeptide domain which provides for an escape of the fusion polypeptide from the phagolysosome into the cytosol of mammalian cells. Preferably, the phagolysosomal escape domain is a Listeria phagolysosomal escape domain, which is described in US 5,733,151, herein incorporated by reference. More preferably, the phagolysosomal escape domain is derived from the listeriolysin gene (*Hly*) of L. monocytogenes. Most preferably, the phagolysosomal domain is encoded by a nucleic acid molecule selected from: (a) a nucleotide sequence comprising nucleotides 211 - 1722 as shown in SEQ ID NO.1, (b) a nucleotide sequence which encodes the same amino acid sequence as the sequence from (a), and (c) a nucleotide sequence hybridizing under stringent conditions with the sequence from (a) or (b).

Apart from the nucleotide sequence depicted in SEQ ID NO.1 the present invention also comprises nucleic acid sequences hybridizing therewith. In the present invention, the term "hybridization" is used as defined in Sambrook et al. (Molecular Cloning. A laboratory manual, Cold Spring Harbor Laboratory Press (1989), 1.101-1.104). In accordance with the present invention the term "hybridization" is used if a positive hybridization signal can still be observed after washing for one hour with 1 × SSC and 0.1 % SDS at 55°C, preferably at 62°C and more preferably at 68°C, particularly for 1 hour in 0.2 × SSC and 0.1 % SDS at 55°C, preferably at 62°C and more preferably at 68°C. A sequence hybridizing with a nucleotide sequence as per SEQ ID No.1 under such washing conditions is a phagolysosomal escape domain encoding nucleotide sequence preferred by the subject invention.

A nucleotide sequence encoding a phagolysosomal escape domain as described above may be directly obtained from a Listeria organism or from any recombinant source e.g., a recombinant *E.coli* cell containing the corresponding Listeria nucleic acid molecule, or a variant thereof as described above.

Preferably, the recombinant nucleic acid molecule encoding for a fusion polypeptide contains a signal peptide encoding sequence. More preferably, the signal sequence is a signal sequence active in Mycobacteria, preferably in *M. bovis,* e.g., a native *M*. *bovis* signal sequence. A preferred example of a suitable signal sequence is the nucleotide sequence coding for the Ag85B signal peptide, which is depicted in SEQ ID NO.1 from nucleotide 1 to 120.

Further, it is preferred that a peptide linker be provided between the immunogenic domain and the phagolysosomal escape domain. Preferably, said peptide linker has a length of from 5 to 50 amino acids. More preferably, a sequence encoding a linker as shown in SEQ ID NO.1 from nucleotide 154 to 210 or a sequence corresponding thereto as regards the degeneration of the genetic code.

The nucleic acid may be located on a recombinant vector. Preferably, the recombinant vector is a prokaryotic vector, i.e., a vector containing elements for replication or/and genomic integration in prokaryotic cells. Preferably, the recombinant vector carries the nucleic acid molecule of the present invention operatively linked with an expression control sequence. The expression control sequence is preferably an expression control sequence active in Mycobacteria, particularly in *M. bovis.* The vector can be an extrachromosomal vector or a vector suitable for integration into the chromosome. Examples of such vectors are known to the man skilled in the art and, for instance, given in Sambrook *et al.* supra.

In particular embodiments, the nucleic acid is integrated into the genome of the Mycobacterium cell, e.g., via homologous recombination in the *ureC* gene.

In particular embodiments, the recombinant Mycobacterium cell comprises a recombinant nucleic acid molecule encoding a fusion polypeptide comprising:
(a) a domain capable of eliciting an immune response comprising the amino acid sequence from aa.41 to aa.51 in SEQ ID NO. 2, and
(b) a Listeria phagolysosomal escape domain encoded by a nucleic acid molecule selected from
   (i) a nucleotide sequence comprising nucleotides 211-1722 as shown in SEQ ID NO. 1,
   (ii) a nucleotide sequence which encodes the same amino acid sequence as the sequence from (i), and
   (iii) a nucleotide sequence hybridizing under stringent conditions with the sequence from (i) or (ii).

In particular embodiments, the *M. bovis* cell is a recombinant urease-deficient *M. bovis* BCG cell from the Danish family strain. Most preferably, the cell is a selection marker-free urease-deficient recombinant BCG Danish family strain characterized as VPM1002 (recombinant BCG Δ*Urec*::*Hly+*)*.* The strain VPM1002 has been classified as BCG Danish subtype Prague strain by standard DNA fingerprinting techniques. According to more advanced DNA sequencing techniques, this classification was substantially confirmed in that VPM1002 is a Danish family strain which falls into DU2 Group 3 (Late Strains-BCG Prague/Danish/Glaxo strains) as per WHO Genealogy of BCG sub-strains (Leung et al., BMC Genomics 9 (2008), 413; Abdallah et al., Scientific Reports 5 (2015), 15443). In particular, VPM1002 was found to be closely related to the BCG Prague strain.

According to the first aspect of the present invention, the vaccine is administered to a human subject which has previously subjected to a treatment against TB.

In certain embodiments of the first aspect, the human subject is an adult, i.e. a person of at least 18 years at the time of vaccination. In particular embodiments of the first aspect, the human subject is an adult of 18-65 years and more particularly an adult of 18-49 years at the time of vaccination. In further embodiments, the human subject is an adolescent, particularly an adolescent with 6-17 years at the time of vaccination.

This aspect of the present invention is directed to the inhibition and/or reduction of TB recurrence, particularly to the inhibition and/or reduction of symptomatic TB recurrence, after a drug treatment of a human subject which previously had been diagnosed with bacteriologically confirmed TB, particularly with bacteriologically confirmed pulmonal TB.

In particular embodiments of the first aspect, the human subject to be vaccinated had been successfully cured by a drug treatment, e.g. as determined by a negative TB sputum smear and culture.

According to the second aspect of the present invention, the vaccine is administered to a human subject which is 6-49 years old. In particular embodiments of the second aspect, the human subject is an adult of 18-49 years at the time of vaccination or an adolescent, and even more particularly an adolescent with 6-17 years at the time of vaccination.

In certain embodiments, the second aspect is directed to the reduction and/or inhibition of TB occurrence, particularly to the inhibition and/or reduction of symptomatic TB occurrence. The term "TB occurrence" as used herein both includes a first occurrence of TB and a recurrence of TB with or without previous drug treatment as described herein.

In certain embodiments of the second aspect, the subject to be vaccinated is a subject having an increased risk of TB occurrence, e.g. a subject living in the same household as a subject which is suffering from TB and/or a subject which has been diagnosed with TB.

In certain embodiments of both aspects, the human subject is preimmunized against TB, e.g., by a previous standard BCG and/or recombinant BCG vaccination.

In certain embodiments, the drug treatment comprises administering at least one antibiotic drug and particularly a combination of antibiotic drugs suitable for the treatment of TB. In particular embodiments, the antibiotic drugs are selected from isoniazid, a rifamycin, e.g., rifampin, rifapentine, or rifabutin, pyrazinamide, ethambutol, a fluoroquinolone, e.g., moxifloxacin, and streptomycin, or any combination thereof. In even more particular embodiments, the treatment is a category 1 TB treatment comprising initially administering isoniazid, rifampin, pyrazinamide and ethambutol and optionally discontinuing ethambutol during the treatment period.

The vaccine is administered to the human subject after the end of the drug treatment, e.g., at least 2 weeks after the end of the drug treatment. In certain embodiments, the vaccine is administered up to 3 months, particularly up to 1 month and more particularly between 2 weeks and 1 month after the end of the drug treatment.

Administration of the vaccine according to the first aspect of the present invention results in a reduction of TB recurrence in previously drug-treated subjects. In certain embodiments, symptomatic TB recurrence is reduced after vaccination, particularly during a period of 2 to 12 months after vaccination. In particular embodiments, symptomatic TB recurrence is reduced by at least 20% or by at least 30% based on the recurrence without vaccination at a time point of 2 months after vaccination. In further embodiments, bacteriologically confirmed TB recurrence is reduced after vaccination, particularly during period 2 to 12 months after vaccination.

Administration of the vaccine according to the second aspect of the present invention results in a reduction and/or inhibition of TB occurrence in subjects which are 18-49 years, e.g., adults of 18-49 years at the time of vaccination or adolescents of 6-17 years at the time of vaccination. In certain embodiments, symptomatic TB occurrence is reduced after vaccination, particularly during a period of period 2 to 12 months after vaccination. In particular embodiments, symptomatic TB occurrence is reduced by at least 20% or by at least 30% based on the occurrence without vaccination at a time point of 2 months after vaccination. In further embodiments, bacteriologically confirmed TB occurrence is reduced after vaccination, particularly during a period 2 to 12 months after vaccination.

The vaccine may be administered as a single dose or in multiple, e.g., 2 or 3 doses. In particular embodiments, a single dose of the vaccine is administered. Typically, the vaccine is administered intradermally, e.g., into a suitable body part, e.g., an arm or an upper leg of the human subject.

The vaccine is administered to the human subject in an effective dose. In particular embodiments, the dose for an administration may be about 10⁵ to 10⁸ viable units (CFU), e.g., about 10⁵-10⁶ CFU, particularly about 2-8 × 10⁵ CFU.

The vaccine is usually provided as a pharmaceutical preparation, which comprises the recombinant Mycobacterial cell in solid form, e.g., a lyophilized or cryo-conserved preparation, which is reconstituted with a suitable liquid carrier before use. Alternatively, the preparation may be provided in liquid form, e.g., as suspension. In certain embodiments, the pharmaceutical preparation comprises excipients including carbohydrates such as dextran and glucose.

### Examples

### 1. Methods

The present study was a double-blind, randomized, placebo-controlled study with two groups of adults (n=1000 per group) receiving either VPM1002 vaccine or placebo. A single dose of VPM1002 or placebo was administered to former TB patients who had successfully completed Category I anti-tubercular treatment (ATT) and were cured. It was conducted at 16 hospitals in India and Bangladesh during January 2018 and December 2023.

### 2. Outcome

The primary objective was to evaluate the efficacy of VPM1002 in prevention of TB recurrence in pulmonary TB patients who had successfully completed ATT and were declared cured. The secondary objective was to evaluate the safety of VPM1002 in this population.

Primary outcome was bacteriologically confirmed recurrence cases at least 2 months after vaccination. The secondary efficacy outcomes were overall recurrence (either bacteriologically confirmed or clinically diagnosed recurrent TB), time to bacteriologically confirmed recurrent TB after at least 2 months from vaccination. The secondary safety outcomes were safety in terms of solicited local and regional reactogenicity within 2 months following study vaccination, unsolicited adverse events and severe adverse events (SAEs) throughout the study period and all-cause mortality.

### 3. Study procedures

Patients were screened up to 25 days after completion of ATT which included acid fast bacteria (AFB) staining and liquid culture of sputum. In eligible participants, vaccination was done 2-4 weeks after ATT completion. The eligible participants were randomized in 1:1 ratio to receive single dose of either VPM1002 or placebo as an intradermal injection. The participants visited the study sites on seven occasions (screening, day 0, 14, month 2, 6, 9 and 12 post vaccination) over a period of around 13 months.

### 4. Efficacy assessment

If a participant developed suspected TB symptoms (including but not limited to persistent cough, haemoptysis, fever, unintended weight loss, fatigue or lethargy, night sweats, or pleuritic chest pain) during the follow-up after vaccination, he/she was advised to report to the study clinic.

Physical examination was done. In case of suspected pulmonary TB, at least one sputum sample was collected for Acid Fast-Bacteria (AFB) staining and liquid culture. In case of suspected extrapulmonary TB, at least one sputum sample and at least one sample from the anatomical site of suspected active TB was collected for AFB staining and liquid culture.

If AFB staining and/or culture was positive, then the participant was referred to ATT and was labelled as a bacteriologically confirmed recurrent TB. If the Investigator decided to put the participant on ATT, even if AFB staining and culture was negative, then the participant was labelled as a clinically diagnosed recurrent TB and was referred to ATT. If both AFB staining and culture were negative, then the participant was followed up till the end of the study.

Whenever there was a difficulty in collecting sputum sample during study period, it was collected by induction.

If recurrent TB was diagnosed within 2 months of vaccination, such participant was withdrawn from the study, and yet followed up for safety until the month 2 visit.

Even if any participant was diagnosed with recurrence TB outside the study clinic based on AFB staining or liquid culture or GenXpert was also considered as bacteriologically confirmed TB recurrence case. All cases of clinically diagnosed TB were reviewed by an independent, blinded endpoint committee to confirm diagnosis.

### 5. Immunogenicity evaluation

In a subset of study population blood for transcriptome analysis and immunological testing was collected at baseline, 2 week, month 2 and at month 12 or at diagnosis of recurrent TB.

### 6. Safety evaluation

Safety assessments included solicited reactions: local (pain, erythema, swelling, ulcer, subcutaneous abscess) and regional (regional lymphadenopathy), unsolicited events and serious adverse events (SAEs). Solicited reactions were collected by structured diaries for 2 months after vaccination while participants reported unsolicited AEs and SAEs throughout the study.

An independent Data Safety Monitoring Board (DSMB) was established to monitor the conduct of the trial. The DSMB periodically examined vaccine safety and provided recommendations regarding continuation of the study.

The first DSMB meeting was convened after the first 200 participants were vaccinated and followed up for at least 2 months for safety. This descriptive safety data was reviewed by the drugs controller general of India (DCGI) and DSMB. After their recommendation, the study was continued.

### 7. Study population

The participants were adults aged 18 to 65 years with bacteriologically confirmed Category I pulmonary TB patients (including controlled diabetics with Hb1Ac level ≤ 7% and non-diabetics) who successfully completed ATT as per the national guidelines and who were cured on the basis of a negative TB sputum smear and culture.

If the sputum liquid culture report from screening (which was available 4-6 weeks after vaccination) was positive, then such participant was labelled as inappropriate enrolment and followed up for safety till 2 months from vaccination. He/she was referred for treatment as per national guidelines. Any of the following excluded an individual from the study: reactive serology for HIV, multidrug-resistant or extensively-drug resistant TB, extrapulmonary TB, immunocompromised status, severe alcohol or illicit drug abuse during past 6 months, pregnant or lactating women and current administration of anti-TB drugs.

### 8. Study vaccines

VPM1002 is a genetically modified *Mycobacterium bovis* BCG from the Danish family strain (rBCGΔ*ureC*:*Hly*+) available as a freeze-dried vaccine with sterile water for injection as a diluent. The excipients include dextran and glucose. A single dose contains 2-8 × 10⁵ CFU VPM1002 administered in 0.1 ml reconstituted solution. VPM1002 is sensitive to antibiotics commonly used in treatment of mycobacterial infection, i.e. isoniazid, rifampicin and ethambutol.

Placebo was a freeze-dried powder containing dextran, glucose and water for injection as diluent.

Both products were stored at 2°C to 8°C and were administered intradermally in the deltoid aspect of the arm.

### 9. Blinding and randomization

The study was double-blinded which means the study participants, investigator, study staff, sponsor and CRO (except unblinded statistician and unblinded data manager) were blinded to treatment assignment.

A permuted block design with random block sizes was used to ensure treatment group balance within each block at each site. Randomization was stratified by site. Treatment assignment occurred through Advantage eClinical^{SM}.

### 10. Statistical analysis

Assuming a 5% recurrence rate in the placebo arm within the 12 months post vaccination and a 50% VE of VPM1002, approximately 2000 participants (1000 in each arm) were required to achieve 80% power at a 5% significance level. This calculation is based on a two-sided Z-test with pooled variance and accounts for a 10% drop-out rate, which includes participants with a positive baseline culture result and with recurrence within the first 2 months.

Modified Intention-To-Treat (mITT) Population included all participants who were randomized and vaccinated, did not develop recurrent TB within the first 2 months after vaccination, completed at least 2 months of follow-up after vaccination, and were baseline culture negative. This was the primary analysis population for the primary and secondary efficacy endpoints. Per Protocol (PP) population comprised of all participants in the mITT population with no major protocol violations. All participants for whom sputum sample were not collected at Month 12 or the sputum AFB or liquid culture result were not available at Month 12 were excluded from PP population. Safety population included all participants who were randomized and vaccinated and was the primary population for safety endpoints.

A logistic regression model was used to assess the primary endpoint of whether a reduction in bacteriologically confirmed recurrence rates is observed in the VPM1002 arm compared to placebo arm. The analysis was adjusted for pooled site, diabetes status at baseline, and presence of previous BCG scar at baseline. Odds ratio and its 95% confidence interval was constructed and evaluated for direction of change in recurrence rate in the VPM1002 arm compared to placebo arm. VE and the corresponding 95% CI were reported in percentages.

Time to event analysis for bacteriologically confirmed recurrence cases was performed using Cox proportional hazards model. The model was adjusted for treatment (VPM1002 or Placebo), pooled site, presence or absence of diabetes at baseline, and presence or absence of previous BCG scar at baseline. All covariates were considered as categorical variables.

Five *post hoc* analyses were conducted for efficacy after the study was unblinded: VE against bacteriologically confirmed symptomatic TB recurrence cases, VE against all symptomatic TB recurrence cases (bacteriologically confirmed + clinically diagnosed), VE against bacteriologically confirmed symptomatic TB Recurrence cases in participants < 50 years of age and VE against all symptomatic TB recurrence cases (bacteriologically confirmed + clinically diagnosed) in participants < 50 years of age and VE analyses were performed in a mITT population 2 (all participants who were randomized and vaccinated, did not develop recurrent TB within the first 1 month after vaccination, completed at least 1 month of follow-up after vaccination, and were baseline culture negative were included in this population).

Safety data was summarized by treatment arm using descriptive statistics. The number and proportion of participants who experienced AEs and the number of AEs was summarized.

All statistical analyses were performed using SAS^{®} software Version 9.4 or later. Medical history and AEs were coded using MedDRA dictionary. Prior and concomitant medications was coded as per WHO DD.

### 11. Ethical considerations

The study was started after the approval from the local regulatory authorities and the Institutional Ethics Committee (IEC) from the respective study centres. A written informed consent was obtained from all the study participants prior to screening. The study followed the Good Clinical Practice guidelines, local regulatory rules and the Declaration of Helsinki.

### 12. Results

A total of 2450 participants were screened and 2001 were randomized to the study. A total 1995 received the study vaccination and constitute the intention-to-treat (ITT) population. The remaining 6 randomized participants (4 from VPM1002 arm and 2 from Placebo arm) discontinued the study prior to vaccination. Three participants received the wrong treatment. Two participants were assigned to VPM1002 arm but received Placebo and one participant was assigned to Placebo arm but received VPM1002. Therefore, out of the 1995 vaccinated participants, 994 (49.8%) received Placebo and 1001 (50.2%) received VPM1002 and constitutes the safety population. The mITT population included 1945 participants, while the PP population included 1863 participants. The mean age was 35.3 years with 55% males. 1696 (84.8%) participants were enrolled in India and 305 (15.2%) in Bangladesh. A total 4.3% were smokers, 3.4% had co-morbidities, 1.4% were diabetics and 55.6% had a previous BCG scar present. The baseline characteristics were similar between the groups.

In the mITT population, 3% (29/979) participants were diagnosed with bacteriologically confirmed TB recurrence in the vaccine group and 3.2% (31/966) participants in the placebo group. The VE was 7.8% (NE, 45.1). In the PP population, TB recurrence occurred in 3.1% (29/934) participants in the Vaccine group and 3.3% (31/929) participants in the Placebo Group. The VE was 7.9% (NE, 45.1). Time to event analysis for bacteriologically confirmed recurrence cases also showed a similar trend. For the secondary outcome of overall TB recurrence cases (including bacteriologically confirmed and clinically diagnosed), there were 3% (29/979) cases in the vaccine group and 3.4% (33/966) cases in placebo group, giving a VE of 13.5% (NE, 48.1). In those with a previous BCG scar, the VE was 20.6% (NE, 58.9) for the bacteriologically confirmed cases while the same was 24.5% (NE, 60.6) for the overall TB recurrence.

*Post hoc* analyses were conducted. In mITT population, for bacteriologically confirmed symptomatic TB recurrence cases, the VE was 25.4% (NE, 59.4%). For overall symptomatic TB recurrence cases (bacteriologically confirmed and clinically diagnosed), the VE was 31% (NE, 62%). In case of participants 18 to 49 years of age, the vaccine showed a VE of 34% (NE, 66.7%) for bacteriologically confirmed symptomatic TB recurrence case and 39.9% (NE, 69.4%) for all symptomatic TB recurrence cases.

In mITT 2 population, the VE was 26.1% (NE, 56.9) for bacteriologically confirmed symptomatic TB recurrence and 30.5% (NE, 59.2) for overall symptomatic TB recurrence. In 18 to 49 years age group for mITT 2 population, the VE was 39.6% (NE, 67.2%) for bacteriologically confirmed symptomatic TB recurrence and 43.7% (NE, 69.3%) for all symptomatic TB recurrence.

Solicited reactions were reported by 64.8% (649/1001) participants in the vaccine group and 15% (149/994) in the placebo group. The common solicited reactions were pain (64.3% and 15%), erythema (5.3% and 0.1%) and swelling (4.7% and 0.1%). Abscess developed in 1% and none in the vaccine and placebo groups, respectively while scar developed in 83.5% and 3.8%. The solicited regional lymphadenopathy was reported by 0.3% and 0.2% of the respective groups.

A total of 23.6% (236/1001) and 23.1% (230/994) participants in the vaccine and placebo group respectively reported unsolicited AEs. Only two events (axillary pain and fever) in one participant from the VPM1002 group were causally related. Both these events were mild in intensity and resolved without any sequalae.

A total 33 SAEs were reported in the study. In the vaccine group 1.8% (18/1001) participants reported 19 SAEs and 1.4% (14/994) participants in the placebo group reported 14 SAEs, and none of them were caused by the study products (Appendix X). Six deaths were reported, 1 in the vaccine group and 5 in the placebo group. The events that led to death were septic shock, myocardial infarction, suicide, congestive cardiac failure and one case each of death unknown and all were causally unrelated to the study products.

### 13. Discussion

This is the first efficacy result for VPM1002 for any TB condition. The double-blind randomized controlled study was conducted in 2001 cured TB patients. The vaccine showed a trend for efficacy against the symptomatic TB recurrence cases, in the overall population as well as in the population of 18 to 49 years of age. The vaccine was safe and well tolerated.

While we assumed for 50% efficacy of the vaccine in the protocol, we did not achieve this figure. However, though it was not actually planned in the protocol, we looked *post hoc* at the symptomatic cases (both in bacteriologically confirmed as well as clinically diagnosed) and in the overall population as well as in the population of 18 to 49 years. Symptomatic cases are important because transmission of TB occurs via aerosolization of infectious droplet nuclei, which are most efficiently produced by coughing and subclinical TB cases, unless they suffer from unrelated cough due to smoking or respiratory tract infections, are less likely to transmit the bacilli.²⁸ Out of total 60 TB recurrence cases meeting primary endpoint in our study, 44 were symptomatic. On Day 365, there was a systematic collection of sputum samples of all participants as a part of the study procedure. This yielded a total 16 cases of bacteriologically positive TB recurrence and all of them were asymptomatic. Since the symptomatic cases are more important in transmission, we specifically looked at VE for the symptomatic cases. The M72/AS01_{E} vaccine trial also looked at efficacy only against symptomatic TB disease.²⁹

The vaccine was found very safe and did not cause any serious adverse reaction or any hypersensitivity reactions. The incidence of local reactions (pain, swelling, erythema, ulcer, abscess) was significantly higher in the vaccine group than the placebo group. This was expected since both BCG and VPM1002 are known to cause local reactions though VPM1002 is less reactogenic than BCG.^{25,26,27} Though the participants were already infected with TB, all the local reactions, except two events of pain, were mild to moderate in severity.

The study took a long time for enrollment of participants - from 2018 to 2022. There were multiple reasons for this. As per the protocol we had to halt the recruitment after vaccination of initial 200 participants. We started recruitment of remaining 1800 participants only after regulatory and DSMB review of 2-month post-vaccination safety data and their approval. TB is still considered as a stigma and as a result many former patients were not ready to come out and take part in the study. COVID-19 period also affected the recruitment as well as the follow-up visits. Vaccine availability was one reason. Despite these challenges we could complete the enrollment and the study follow-up.

Out of 1995 participants vaccinated in the study, 1872 completed the study. This excludes early terminations due to positive baseline liquid culture (n=17), TB recurrence cases within 2 months of vaccination (n=16), and death (n=6). Thus, one-year retention rate in our study was 93.8%, which was better than the six month anti-TB treatment adherence rate in India of around 84%.³⁰

Adults aged 18 to 49 showed a higher trend of efficacy against TB recurrence as compared to the entire population of 18 years and above. BCG vaccination at birth is effective at preventing TB in young children but is ineffective in adolescents and adults.³¹ Therefore, our results show better trend as compared to BCG. Possible reason for low efficacy in elderly population could be lower immune response though we did not assess the same.

Another TB candidate, H56:IC31 was recently tested for prevention of TB recurrence in a Phase-2b study. However, the vaccine did not provide protection against TB recurrence and in fact, possibly increased risk of recurrence among people who received the study vaccine (5.8%) compared to people who received the placebo (3.4%).³² Our results are better than those of the H56:IC31 candidate.

For all efficacy end-points, the lower bound 95%CI was less than zero indicating statistical non-significance. Despite this, we consider it likely that the results showed a trend for efficacy against symptomatic TB recurrence and more so in the 18 to 49 age group. The follow-up of the study was one year considering the fact that 90% of TB recurrence cases occur within one year of cure.¹¹ Based on the sample size calculation, we required 75 cases of bacteriologically confirmed TB recurrence cases during this period. However, we ended up with 60 such cases, falling short of 15 cases. In hindsight, if we had added one more year of follow-up, we could have reached the required number of cases and the results could have been statistically significant. Any similar trial needs to consider a 2 years follow-up for this indication.

We consider prevention of TB recurrence a particularly challenging target for any TB vaccine candidate. These patients had natural symptomatic TB disease and 5 to 10% of patients show a recurrence of TB within the first few months, indicating that a natural MTB symptomatic disease does not provide adequate protection against subsequent disease. In such a situation it becomes a high bar for any TB vaccine candidate to cross. In fact, this becomes challenging even for other TB vaccine candidates.

The strengths of our study include a very robust design and a meticulous follow-up to capture the cases. As mentioned above, probably the one-year follow-up was insufficient and a limitation. We systematically collected the sputum samples for a year after the vaccination, which detected 16 cases. We could have done this more frequently during the one-year follow-up period which may have detected more such cases. However, any such asymptomatic cases that happened before one year would have been detected even at the one-year point time. Therefore, we do not think this has any impact on the study findings. We also wonder whether symptomatic TB diseases would have been a correct end point for this trial, as was done for the H56:IC31 vaccine and the M72 vaccine in their respective trials.^{19,32}

There were 15 bacteriologically confirmed TB recurrence cases during the first 2 months of vaccination which were not counted towards the end point, as these were probably not completely cured cases. Moreover, we thought it would take at least 2 months for VPM1002 to mount a protective immune response. The cellular immune response to BCG generally peaks at 6-10 weeks.^{33,34} Earlier studies on VPM1002 also suggest that the immune responses peak at 6 weeks after vaccination.^{26,27} *Post hoc* we did look at the cases which happened one month after the vaccination, and the VE increased marginally.

At the time of screening we collected the sputum samples and tested the AFB smear for screening. We also sent the samples for liquid culture. Since it would take at least 4-8 weeks to get the liquid culture results, we enrolled such patients in the study on the basis of negative AFB smear, as waiting for liquid culture results would have delayed vaccination and we might have had lost crucial initial period as most of the TB recurrence cases, particularly relapse happen within first 6 months after completion of the treatment. In our study 16 (0.8%) participants with negative AFB smear turned out positive in the liquid culture for the screening sputum sample indicating that they were not fully cured. They were withdrawn from the study as inappropriate enrollments. Out of 2450 screened participants, 43 (1.8%) were positive for TB either by AFB smear or by liquid culture. One study in Nigeria showed a treatment failure rate of 1.4% in HIV negative patients.³⁵ Our study population was HIV negative.

We kept a gap of minimum 2 weeks from the completion of the ATT and the study vaccination. This was necessary because there should be a washout of the ATT drugs from the body so that they will not affect the action of VPM1002, which is a live attenuated *M. bovis* vaccine. The half-lives of the frontline ATT drugs - INH, rifampicin, ethambutol and pyrazinamide are in the range of approximately less than one hour to 10 hours,³⁶ thus the drugs would be completely washed out of the body in 2 weeks. As mentioned above, VMP1002 is sensitive to all ATT drugs.

We screened the study participants after they had completed their treatments and they were declared as cured. As a result, we could not get their original MTB strain of infection, while we could only get the recurrence TB strain results. In the absence of the original strain, we could not distinguish the recurrence cases into relapses and new infections.

To conclude, VPM1002 was found very safe and was well tolerated in the cured TB patients. The vaccine also showed a strong trend towards prevention of symptomatic TB recurrence, especially in the younger adults, despite the challenges of testing TB vaccine in this indication and overcoming the natural immunity caused by MTB infection. The present study indicates that VPM-1002 can be considered as an option to prevent TB recurrence.

### Sequences

The sequences listed as SEQ ID NO.1 and SEQ ID NO.2 are as follows:
**SEQ ID NO. 1:**
   *Nucleotide sequence encoding a phagolysosomal domain (nt 211-1722) and* a *stop codon (nt 1879-1881)* 
atgacagacg tgagccgaaa gattcgagct tggggacgcc gattgatgat cggcacggca
gcggctgtag tccttccggg cctggtgggg cttgccggcg gagcggcaac cgcgggcgcg
ttctcccggc cggggctgcc ggtcgagtac ctgcagtctg caaagcaatc cgctgcaaat
aaattgcact cagcaggaca aagcacgaaa gatgcatctg cattcaataa agaaaattca
atttcatcca tggcaccacc agcatctccg cctgcaagtc ctaagacgcc aatcgaaaag
aaacacgcgg atgaaatcga taagtatata caaggattgg attacaataa aaacaatgta
ttagtatacc acggagatgc agtgacaaat gtgccgccaa gaaaaggtta caaagatgga
aatgaatata ttgttgtgga gaaaaagaag aaatccatca atcaaaataa tgcagacatt
caagttgtga atgcaatttc gagcctaacc tatccaggtg ctctcgtaaa agcgaattcg
gaattagtag aaaatcaacc agatgttctc cctgtaaaac gtgattcatt aacactcagc
attgatttgc caggtatgac taatcaagac aataaaatcg ttgtaaaaaa tgccactaaa
tcaaacgtta acaacgcagt aaatacatta gtggaaagat ggaatgaaaa atatgctcaa
gcttatccaa atgtaagtgc aaaaattgat tatgatgacg aaatggctta cagtgaatca
caattaattg cgaaatttgg tacagcattt aaagctgtaa ataatagctt gaatgtaaac
ttcggcgcaa tcagtgaagg gaaaatgcaa gaagaagtca ttagttttaa acaaatttac
tataacgtga atgttaatga acctacaaga ccttccagat ttttcggcaa agctgttact
aaagagcagt tgcaagcgct tggagtgaat gcagaaaatc ctcctgcata tatctcaagt
gtggcgtatg gccgtcaagt ttatttgaaa ttatcaacta attcccatag tactaaagta
aaagctgctt ttgatgctgc cgtaagcgga aaatctgtct caggtgatgt agaactaaca
aatatcatca aaaattcttc cttcaaagcc gtaatttacg gaggttccgc aaaagatgaa
gttcaaatca tcgacggcaa cctcggagac ttacgcgata ttttgaaaaa aggcgctact
tttaatcgag aaacaccagg agttcccatt gcttatacaa caaacttcct aaaagacaat
gaattagctg ttattaaaaa caactcagaa tatattgaaa caacttcaaa agcttataca
gatggaaaaa ttaacatcga tcactctgga ggatacgttg ctcaattcaa catttcttgg
gatgaagtaa attatgatcc tgaaggtaac gaaattgttc aacataaaaa ctggagcgaa
 
aacaataaaa gcaagctagc tcatttcaca tcgtccatct atttgccagg taacgcgaga
aatattaatg tttacgctaa agaatgcact ggtttagctt gggaatggtg gagaacggta
attgatgacc ggaacttacc acttgtgaaa aatagaaata tctccatctg gggcaccacg
ctttatccga aatatagtaa taaagtagat aatccaatcg aatatgcatt agcctatgga
agtcagggtg atcttaatcc attaattaat gaaatcagca aaatcatttc agctgcagtt
ctttcctctt taacatcgaa gctacctgca gagttcgtta ggcgcggatc cggaattcga
agcttatcga tgtcgacgta g
**SEQ ID NO. 2:**
   *Amino acid sequence encoded by the nucleotide sequence of SEQ ID NO: 1*

Met Thr Asp Val Ser Arg Lys Ile Arg Ala Trp Gly Arg Arg Leu Met Ile Gly Thr Ala Ala
Ala Val Val Leu Pro Gly Leu Val Gly Leu Ala Gly Gly Ala Ala Thr Ala Gly Ala Phe Ser
Arg Pro Gly Leu Pro Val Glu Tyr Leu Gln Ser Ala Lys Gln Ser Ala Ala Asn Lys Leu His
Ser Ala Gly Gln Ser Thr Lys Asp Ala Ser Ala Phe Asn Lys Glu Asn Ser Ile Ser Ser Met
Ala Pro Pro Ala Ser Pro Pro Ala Ser Pro Lys Thr Pro Ile Glu Lys Lys His Ala Asp Glu
Ile Asp Lys Tyr Ile Gln Gly Leu Asp Tyr Asn Lys Asn Asn Val Leu Val Tyr His Gly Asp
Ala Val Thr Asn Val Pro Pro Arg Lys Gly Tyr Lys Asp Gly Asn Glu Tyr Ile Val Val Glu
Lys Lys Lys Lys Ser Ile Asn Gln Asn Asn Ala Asp Ile Gln Val Val Asn Ala Ile Ser Ser
Leu Thr Tyr Pro Gly Ala Leu Val Lys Ala Asn Ser Glu Leu Val Glu Asn Gln Pro Asp
Val Leu Pro Val Lys Arg Asp Ser Leu Thr Leu Ser Ile Asp Leu Pro Gly Met Thr Asn
Gln Asp Asn Lys Ile Val Val Lys Asn Ala Thr Lys Ser Asn Val Asn Asn Ala Val Asn
Thr Leu Val Glu Arg Trp Asn Glu Lys Tyr Ala Gln Ala Tyr Pro Asn Val Ser Ala Lys Ile
Asp Tyr Asp Asp Glu Met Ala Tyr Ser Glu Ser Gln Leu Ile Ala Lys Phe Gly Thr Ala
Phe Lys Ala Val Asn Asn Ser Leu Asn Val Asn Phe Gly Ala Ile Ser Glu Gly Lys Met
Gln Glu Glu Val Ile Ser Phe Lys Gln Ile Tyr Tyr Asn Val Asn Val Asn Glu Pro Thr Arg
Pro Ser Arg Phe Phe Gly Lys Ala Val Thr Lys Glu Gln Leu Gln Ala Leu Gly Val Asn
Ala Glu Asn Pro Pro Ala Tyr Ile Ser Ser Val Ala Tyr Gly Arg Gln Val Tyr Leu Lys Leu
Ser Thr Asn Ser His Ser Thr Lys Val Lys Ala Ala Phe Asp Ala Ala Val Ser Gly Lys Ser
Val Ser Gly Asp Val Glu Leu Thr Asn Ile Ile Lys Asn Ser Ser Phe Lys Ala Val Ile Tyr
Gly Gly Ser Ala Lys Asp Glu Val Gln Ile Ile Asp Gly Asn Leu Gly Asp Leu Arg Asp Ile
Leu Lys Lys Gly Ala Thr Phe Asn Arg Glu Thr Pro Gly Val Pro Ile Ala Tyr Thr Thr Asn
Phe Leu Lys Asp Asn Glu Leu Ala Val Ile Lys Asn Asn Ser Glu Tyr Ile Glu Thr Thr Ser
Lys Ala Tyr Thr Asp Gly Lys Ile Asn Ile Asp His Ser Gly Gly Tyr Val Ala Gln Phe Asn
Ile Ser Trp Asp Glu Val Asn Tyr Asp Pro Glu Gly Asn Glu Ile Val Gln His Lys Asn Trp
Ser Glu Asn Asn Lys Ser Lys Leu Ala His Phe Thr Ser Ser Ile Tyr Leu Pro Gly Asn
Ala Arg Asn Ile Asn Val Tyr Ala Lys Glu Cys Thr Gly Leu Ala Trp Glu Trp Trp Arg Thr
Val Ile Asp Asp Arg Asn Leu Pro Leu Val Lys Asn Arg Asn Ile Ser Ile Trp Gly Thr Thr
Leu Tyr Pro Lys Tyr Ser Asn Lys Val Asp Asn Pro Ile Glu Tyr Ala Leu Ala Tyr Gly Ser
Gln Gly Asp Leu Asn Pro Leu Ile Asn Glu Ile Ser Lys Ile Ile Ser Ala Ala Val Leu Ser
Ser Leu Thr Ser Lys Leu Pro Ala Glu Phe Val Arg Arg Gly Ser Gly Ile Arg Ser Leu Ser
Met Ser Thr

### References:

1. https://www.who.int/news-room/fact-sheets/detail/tuberculosis#- --text=Tuberculosis%20(TB)%20is%20an%20infe ctious,been%20infected%20with%20TB%20bacteria. Accessed on 29 October 2024.
2. Mandal S, Rao R, Joshi R. Estimating the Burden of Tuberculosis in India: A Modelling Study. Indian J Community Med. 2023 May-Jun;48(3):436-442.
3. Selvaraju S, Velayutham B, Rao R, et al. Prevalence and factors associated with tuberculosis infection in India. J Infect Public Health. 2023 Dec;16(12):2058-2065.
4. Bangladesh tuberculosis roadmap overview, Fiscal year 2023. https://www.usaid.gov/sites/default/files/2024-01/Banaladesh Road map 2023 narrative for web 508.pdf Accessed on 29 October 2024
5. Implementing the WHO Stop TB Strategy: A Handbook for National Tuberculosis Control Programmes. Geneva: World Health Organization; 2008. 2, Treatment of tuberculosis patients. Available from: https://www.ncbi.nlm.nih.gov/books/NBK310759/
6. Directorate General of Health Services. New Delhi: MOHFW Gol; 2009. Central TB Division, TB India 2009 RNTCP status report, in TB India.
7. Cox HS, Morrow M, Deutschmann PW. Long term efficacy of DOTS regimens for tuberculosis: Systematic review. Br Med J. 2008;336:484-7.
8. Thomas A, Gopi PG, Santha T. et al. Predictors of relapse among pulmonary tuberculosis patients treated in a DOTS programme in South India. Int J Tuberc Lung Dis. 2005;9:556-61.
9. Mehra RK, Dhingra VK, Nish A, Vashist RP. Study of relapse and failure cases of CAT I retreated with CAT II under RNTCP-an eleven year follow up. Indian J Tuberc. 2008;55:188-91.
10. Gulrez S.A. DOTS for TB relapse in India: A systematic review. Lung India. 2012; 29(2): 147-153.
11. Millet J-P, Shaw E, Orcau A', et al. Tuberculosis Recurrence after Completion Treatment in a European City: Reinfection or Relapse? PLoS ONE. 2013;8(6): e64898
12. Kasozi S, Clark J, Doi SA. Intermittent Versus Daily Pulmonary Tuberculosis Treatment Regimens: A Meta-Analysis. Clin Med Res. 2015 Dec; 13(3-4):117-38.
13. Gopalan N, Santhanakrishnan RK, Palaniappan AN, et al. Daily vs Intermittent Antituberculosis Therapy for Pulmonary Tuberculosis in Patients With HIV: A Randomized Clinical Trial. JAMA Intern Med. 2018 Apr 1;178(4):485-493.
14. Bose A, Kalita S, Rose W, Tharyan P. Intermittent versus daily therapy for treating tuberculosis in children. Cochrane Database Syst Rev. 2014 Jan 28;2014(1):CD007953.
15. Johnston JC, Campbell JR, Menzies D. Effect of Intermittency on Treatment Outcomes in Pulmonary Tuberculosis: An Updated Systematic Review and Metaanalysis. Clin Infect Dis. 2017 May 1;64(9):1211-1220.
16. Health Ministry introduces Daily Drug Regimen for treatment of Tuberculosis. Posted On: 17 NOV 2017. https://pib.gov.in/PressReleasePage.aspx?PRID=1509968 Accessed on 31 October 2024.
17. Sahadevan R, Narayanan S, Paramasivan CN, Prabhakar R, Narayanan PR. Restriction fragment length polymorphism typing of clinical isolates of Mycobacterium tuberculosis from patients with pulmonary tuberculosis in Madras, India, by use of direct-repeat probe. J Clin Microbiol. 1995 Nov;33(11):3037-9.
18. De S. High relapse rate in RNTCP: An increasing concern and time to intervene. Lung India. 2013 Jan;30(1):85-6.
19. Mangtani P., Abubakar I., Ariti C., Beynon R., Pimpin L., Fine P.E.M. Protection by BCG vaccine against tuberculosis: a systematic review of randomized controlled trials. Clin. Infect. Dis. 2014;58(4):470-480.
20.Andersen, P. 2007. Tuberculosis vaccines - an update. Nat.Rev.Microbiol. 5:484-487.
21.Mittrücker HW, Steinhoff U, Köhler A, et al. Poor correlation between BCG vaccination-induced T cell responses and protection against tuberculosis. Proc Natl Acad Sci USA. 2007 Jul 24; 104(30): 12434-9.
22. Schaible UE, Winau F, Sieling PA, et al. Apoptosis facilitates antigen presentation to T lymphocytes through MHC-I and CD1 in tuberculosis. Nat Med. 2003 Aug;9(8):1039-46.
23. Grode L, Seiler P, Baumann S, et al. Increased vaccine efficacy against tuberculosis of recombinant Mycobacterium bovis bacille Calmette-Guérin mutants that secrete listeriolysin. J Clin Invest. 2005 Sep;115(9):2472-9.
24. Gengenbacher M, Kaiser P, Schuerer S, Lazar D, Kaufmann SH. Post-exposure vaccination with the vaccine candidate Bacillus Calmette-Guérin ΔureC::hly induces superior protection in a mouse model of subclinical tuberculosis. Microbes Infect. 2016 May;18(5):364-8.
25. Grode L, Ganoza CA, Brohm C, Weiner J 3rd, Eisele B, Kaufmann SH. Safety and immunogenicity of the recombinant BCG vaccine VPM1002 in a phase 1 open-label randomized clinical trial. Vaccine. 2013 Feb 18;31(9):1340-8.
26. Loxton AG, Knaul JK, Grode L, et al. Safety and Immunogenicity of the Recombinant Mycobacterium bovis BCG Vaccine VPM1002 in HIV-Unexposed Newborn Infants in South Africa. Clin Vaccine Immunol. 2017 Feb 6;24(2):e00439-16.
27. Cotton MF, Madhi SA, Luabeya AK, et al. Safety and immunogenicity of VPM1002 versus BCG in South African newborn babies: a randomised, phase 2 non-inferiority double-blind controlled trial. Lancet Infect Dis. 2022 Oct;22(10):1472-1483.
28. Esmail H, Dodd PJ, Houben RMGJ. Tuberculosis transmission during the subclinical period: could unrelated cough play a part? Lancet Respir Med. 2018 Apr;6(4):244-246.
29. Tait DR, Hatherill M, Van Der Meeren O, et al. Final Analysis of a Trial of M72/AS01E Vaccine to Prevent Tuberculosis. N Engl J Med. 2019 Dec 19;381(25):2429-2439.
30. Bagchi S, Ambe G, Sathiakumar N. Determinants of poor adherence to antituberculosis treatment in mumbai, India. Int J Prev Med. 2010 Fall;1(4):223-32.
31. Martinez L, Cords O, Liu Q, et al. Infant BCG vaccination and risk of pulmonary and extrapulmonary tuberculosis throughout the life course: a systematic review and individual participant data meta-analysis. Lancet Glob Health. 2022 Sep;10(9):e1307-e1316.
32. Development of the Candidate Tuberculosis Vaccine H56:IC31 Ended Based on Early Data from the Prevention of Recurrence (POR) TB Consortium. Updated 19 December 2023. https://www.porconsortium.org/news/2023/early-results-on-the-a-055-por-trial Accessed on 21 October 2024.
33. Soares AP, Kwong Chung CK, Choice T, et al. Longitudinal changes in CD4(+) T-cell memory responses induced by BCG vaccination of newborns. J Infect Dis. 2013 Apr;207(7):1084-94.
34. Kagina BM, Abel B, Bowmaker M, et al. Delaying BCG vaccination from birth to 10 weeks of age may result in an enhanced memory CD4 T cell response. Vaccine. 2009 Sep 4;27(40):5488-95.
35. Ukwaja KN, Oshi SN, Alobu I, Oshi DC. Profile and determinants of unsuccessful tuberculosis outcome in rural Nigeria: Implications for tuberculosis control. World J Methodol. 2016 Mar 26;6(1):118-25.
36. Treatment of Tuberculosis: Guidelines. 4th edition. Geneva: World Health Organization; 2010. A1, Essential first-line antituberculosis drugs. Available from: https://www.ncbi.nlm.nih.gov/books/NBK138747/ Accessed on 12 November 2024.
37. Singh M, Mehendale S, Guleria R, et al. PreVenTB trial: protocol for evaluation of efficacy and safety of two vaccines VPM1002 and Immuvac (Mw) in preventing tuberculosis (TB) in healthy household contacts of newly diagnosed sputum smear-positive pulmonary TB patients: phase III, randomised, double-blind, three-arm placebo-controlled trial. BMJ Open 2024;14:e082916. doi:10.1136/bmjopen-2023-082916

## Claims

1. A recombinant urease-deficient *M. bovis* BCG cell from the Danish family strain, which comprises a recombinant nucleic acid molecule encoding a fusion polypeptide comprising:
(a) a domain capable of eliciting an immune response, and
(b) a phagolysosomal escape domain
for use in a method, wherein the recombinant BCG cell is administered as a vaccine to a human subject which has previously been subjected to a drug treatment against TB.

2. A recombinant urease-deficient *M. bovis* BCG cell from the Danish family strain, which comprises a recombinant nucleic acid molecule encoding a fusion polypeptide comprising:
(a) a domain capable of eliciting an immune response, and
(b) a phagolysosomal escape domain
for use in a method, wherein the recombinant BCG cell is administered as a vaccine to a human subject which is 6-49 years old.

3. The recombinant Mycobacterium cell of claim 1 for the use of claim 1 or 2, wherein the recombinant nucleic acid molecule encoding a fusion polypeptide comprises:
(a) a domain capable of eliciting an immune response comprising the amino acid sequence from aa.41 to aa.51 in SEQ ID NO.2, and
(b) a Listeria phagolysosomal escape domain encoded by a nucleic acid molecule selected from
(i) a nucleotide sequence comprising nucleotides 211-1722 as shown in SEQ ID NO.1,
(ii) a nucleotide sequence which encodes the same amino acid sequence as the sequence from (i), and
(iii) a nucleotide sequence hybridizing under stringent conditions with the sequence from (i) or (ii).

4. The cell of any one of claims 1-3 for the use of claim 1 or 2, which is the BCG strain VPM1002 *(rBCGΔureC::Hly+).*

5. The cell of any one of claims 1-4 for the use of claim 1 or 2, wherein the human subject is an adult of 18-49 years, or wherein the human subject is an adolescent, particularly an adolescent with 6-17 years.

6. The cell of any one of claims 1-4 for the use of claim 1 or 5, wherein the human subject was diagnosed with bacteriologically confirmed TB, particularly with bacteriologically confirmed pulmonal TB before the drug treatment.

7. The cell of any one of claims 1-4 for the use of any one of claims 1 or 5-6, wherein the human subject had been successfully cured by the drug treatment.

8. The cell of any one of claims 1-4 for the use of any one of claims 1 or 5-7, wherein the human subject had been treated with at least one antibiotic drug, particularly with a combination of antibiotic drugs, wherein the antibiotic drugs are particularly selected from isoniazid, a rifamycin, e.g., rifampin, rifapentine, or rifabutin, pyrazinamide, ethambutol, and a fluoroquinolone, e.g., moxifloxacin or any combination thereof.

9. The cell of any one of claims 1-4 for the use of any one of claims 1, 2 or 5-8, wherein the vaccine is administered up to 3 months, particularly up to 1 month and more particularly between 2 weeks and 1 month after the end of the drug treatment.

10. The cell of any one of claims 1-4 for the use of any one of claims 1 or 5-9, wherein bacteriologically confirmed TB recurrence is reduced after vaccination, particularly during a period of 2 to 12 months after vaccination, and particularly wherein symptomatically confirmed TB recurrence is reduced after vaccination, particularly during a period of period 2 to 12 months after vaccination.

11. The cell of any one of claims 1-4 for the use of any one of claims 2 or 5-10, wherein bacteriologically confirmed TB occurrence is reduced after vaccination, particularly during a period of 2 to 12 months after vaccination, and particularly wherein symptomatically confirmed TB occurrence is reduced after vaccination, particularly during a period of period 2 to 12 months after vaccination.

12. The cell of any one of claims 1-4 for the use of any one of claims 1, 2 or 5-11, wherein a single dose of the vaccine is administered.

13. The cell of any one of claims 1-4 for the use of any one of claims 1, 2 or 5-12, wherein a dose of the vaccine comprises about 10⁵-10⁶ CFU, particularly about 2-8 × 10⁵ CFU.

14. The cell of any one of claims 1-4 for the use of any one of claims 1, 2 or 5-13, wherein the human subject is preimmunized against TB, e.g., by a previous standard BCG and/or recombinant BCG vaccination.

15. A method of vaccinating a human subject against TB, comprising administering to said subject an effective amount of a live Mycobacterium cell which comprises a recombinant nucleic acid molecule encoding a fusion polypeptide comprising:
(a) a domain capable of eliciting an immune response, and
(b) a phagolysosomal escape domain,
wherein the human subject has previously been subjected to a drug treatment against TB and/or wherein the human subject is 6-49 years old.
